# EUROPEAN PATENT SPECIFICATION

(11) **EP 4 731 283 B1**
(45) Date of publication and mention of the grant of the patent: **02.09.2026**
(21) Application number: 24752185.9
(22) Date of filing: 09.07.2024
(51) Int. Cl.: A61M 5/20, A61B 5/145, A61B 5/332

(54) **BIO-ELECTRICAL SENSING IN AN AUTOINJECTOR**
BIOELEKTRISCHE MESSUNG IN EINEM AUTOINJEKTOR
DÉTECTION BIOÉLECTRIQUE DANS UN AUTO-INJECTEUR

(30) Priority: 13.07.2023 US 202363526530 P; 29.03.2024 US 202418621862
(43) Date of publication of application: 29.04.2026
(73) Proprietor: Flextronics AP, LLC, San Jose, CA 95002 (US)
(72) Inventor: DE ANGELI, Marco, 24030 Barzana (IT); ORLANDO, Riccardo, 47522 Cesena (IT); FORTE, Salvatore, 20147 Milano (IT)
(74) Representative: FRKelly
(86) International application number: PCT/US2024/037164
(87) International publication number: WO 2025/014927

(56) References cited:
- EP-B1- 1 501 405
- CA-A1- 2 930 740
- US-A1- 2023 201 463
- US-B1- 10 610 111
- US-B2- 10 105 489

## Description

### FIELD OF THE INVENTION

The present disclosure is directed to an autoinjector medical device, and more particularly it is directed to bio-electrical sensing in an autoinjector medical device.

### BACKGROUND OF THE INVENTION

An autoinjector medical device is a device that is configured to deliver a dose of a particular drug or medication into a user. Autoinjectors were designed to overcome the hesitation associated with self-administration of needle-based drug delivery. Most autoinjectors utilize one-use, disposable syringes that are prefilled with the drug to be delivered. After the drug has been administered, the syringe can be disposed of and a new prefilled syringe can be inserted into the autoinjector for the next drug delivery. Real-time monitoring of physiological parameters of a user or patient at the point of care is currently one of the major trends in the healthcare industry. It represents an opportunity for innovation in portable medical devices, particularly in the drug delivery sector. As such, there is a desire to incorporate real-time monitoring of physiological parameters of a user or patient into an autoinjector medical device.

EP 1 501 405 B1 relates to a doser comprising a syringe with a needle which extends beyond the doser, which comprises an engagement face in the vicinity of the needle so that the engagement face rests against the surface of the tissue into which the needle is inserted. Detector means are provided on said engagement face to sample signals on the skin of the patient, said means being arranged for providing heart rate signals. The disclosure further provides means for receiving information related to health monitoring of a patient. This provides a doser that may record heart rate, EKG, BGM and hypo-alarm administered medicine. The doser may further be arranged to calculate an appropriate dose of medication on the basis of a number of acquired inputs.

US 10,610,111 B1 discloses a system featuring a wearable device designed to monitor a user's movement, mood, and vital signs. The system includes an accelerometer for motion sensing, various physiological sensors with at least one on the back of the device, and multiple wireless transceivers for cellular, WiFi, and Bluetooth connectivity.

US 2023/0201463 describes a medicament delivery system which can include a smart user device and a medicament delivery assembly. The medicament delivery assembly can be implemented as a single-use cartridge that can be removably coupled to the smart user device. The smart user device can be configured to determine when to automatically administer a medicament stored in the medicament delivery assembly.

CA 2930740 A1 relates to systems, methods and apparatuses for monitoring cardiac activity of an individual using a conformal cardiac sensor device. A conformal cardiac sensor device for analyzing cardiac activity includes a flexible substrate for coupling to the user, and a heart sensor component embedded on/in the substrate. The heart sensor component contacts a portion of the users skin and measures electrical variable(s) indicative of cardiac activity. A biometric sensor component is embedded on/in the flexible substrate and measures physiological variable(s) indicative of cardiac activity of the user. A microprocessor, which is embedded on/in the flexible substrate, is communicatively coupled to the heart sensor component and biometric sensor component and operable to execute microprocessor executable instructions for controlling the measurements of electrical data and physiological data. A wireless communication component is embedded on/in the flexible substrate and is operable to transmit data indicative of the measurements obtained by the sensor components.

US 10,105,489 describes a system which includes a medicament delivery device and a container configured to receive at least a portion of the medicament delivery device. The medicament delivery device includes an actuator and a first electronic circuit system. The actuator is configured to initiate delivery of a medicament into a body when the actuator is moved from a first position to a second position. The first electronic circuit system is configured to output a first electronic signal when the actuator is moved from the first position to the second position. The container includes a second electronic circuit system configured to receive the first electronic signal. The second electronic circuit system is configured to output a second electronic signal associated with the first electronic signal.

### SUMMARY OF THE INVENTION

The invention is defined by the subject-matter of claims 1 and 10. Further embodiments are defined in the dependent claims. According to one aspect, an autoinjector medical device is disclosed. The autoinjector medical device can include a cover including: an engaging surface including a needle outlet, and a handle formed in the cover at an opposite end of the cover as the engaging surface. Further, the autoinjector medical device can include a needle configured to translate through the needle outlet, a first sensor electrode disposed on the engaging surface and adjacent the needle outlet, and a second sensor electrode disposed on the handle. The first sensor electrode and the second sensor electrode can be configured for biometric authentication of a user, wherein the device further comprises stored identity data and is configured to compare sensed parameters of the user to the stored identity data to verify the identity of the user before allowing an injection.

In one aspect, the autoinjector medical device can further include a third electrode disposed on the handle.

In one aspect, the third electrode is positioned on an opposite side of the handle as the second sensor electrode.

In one aspect, the third electrode is a right-leg drive electrode configured to implement common-mode powerline interference suppression.

According to the invention, the first sensor electrode and the second sensor electrode are each electrode sensors configured to measure and record electrical signals produced by a heart or body of a user.

In one aspect, the first sensor electrode and the second sensor electrode are configured to measure and record electrical signals produced by the heart of the user before, during, or after insertion of the needle into the user.

In one aspect, the first sensor electrode and the second sensor electrode are configured to measure and record one or more of heart rate, heart rate variability, physiological stress, energy expenditure, atrial fibrillation, arrhythmia, body composition (e.g., body fat mass), and hydration level (i.e., body water) in the user.

In one aspect, the autoinjector medical device can further include an alarm configured to alert the user when the measured and record electrical signals exceed a pre-defined threshold limit.

In one aspect, the first sensor electrode and the second sensor electrode can further be configured for at least one of biometric authentication and bio-impedance analysis of the user.

In one aspect, the autoinjector medical device can further include an analog front-end stage configured to precondition the electrical signals measured and recorded by the first sensor electrode and the second sensor electrode before processing and outputting results regarding the electrical signals.

In one aspect, the autoinjector medical device is an electro-mechanical autoinjector medical device including a power source configured to supply electrical energy to an actuator, the actuator being coupled to the needle and the actuator being configured to cause the needle to translate through the needle outlet.

In one aspect, the autoinjector medical device can further include a display disposed on or coupled to the cover, the display being configured to provide a visual presentation of data collected by the autoinjector medical device.

In one aspect, the autoinjector medical device can further include an input-output device configured to transfer data collected by the autoinjector medical device to a device separate and remote from the autoinjector medical device.

In one aspect, the input-output device can transfer collected data through one or more of a wireless network protocol (Wi-Fi), a cellular signal, a Bluetooth standard, and a cloud-based data transfer service.

According to another aspect, a method of collecting electrical signals of a heart or body of a user with an autoinjector medical device is disclosed. The method can include grasping a handle of the autoinjector medical device with a hand on a first side of a user's body, wherein during the grasping a second sensor electrode of the autoinjector medical device directly contacts the hand of the user; pressing an engaging surface of the autoinjector medical device directly against a portion of the user's body on a second side of the user's body, wherein during the pressing a first sensor electrode of the autoinjector medical device directly contacts the portion of the second side of the user's body; and measuring and collecting, by the first sensor electrode and the second sensor electrode, electrical signals produced by the heart or body of the user; comparing the collected electrical signals to stored identity data to verify the identity of the user; and allowing an injection of a drug from the autoinjector medical device only upon verification of the identity of the user.

In one aspect, the method can further include directly contacting the first sensor electrode and the second sensor electrode to skin surfaces of the user's body.

In one aspect, the first side of the user's body and the second side of the user's body are positioned on opposite sides of a sagittal plane of the user's body.

In one aspect, the method can further include processing, by an analog front-end stage of the autoinjector medical device, the measured and collected electrical signals, wherein the processing comprises filtering the electrical signals to remove common-mode powerline interferences.

In one aspect, the method can further include converting, by an analog to digital converter of the autoinjector medical device, the processed electrical signals into digital data; and processing and outputting, by a microprocessor of the autoinjector medical device, physiological parameters relating to the heart or body of the user.

In one aspect, the method can further include displaying the physiological parameters on a display of the autoinjector medical device; or communicating the physiological parameters through an input-output device of the autoinjector medical device to a device separate and remote from the autoinjector medical device.

### BRIEF DESCRIPTION OF THE DRAWINGS

The foregoing Summary as well as the following Detailed Description will be best understood when read in conjunction with the appended drawings, which illustrate a preferred embodiment of the disclosure. In the drawings:
FIG. 1 is a perspective front-view of an autoinjector medical device according to the present disclosure.
FIG. 2 is a rear view of the autoinjector medical device of FIG. 1, illustrating internal componentry of the autoinjector medical device.
FIG. 3 is a perspective view of a user holding and operating the autoinjector medical device.
FIG. 4 is another view of a user holding and operating the autoinjector medical device.
FIG. 5 is a schematic block diagram illustrating method steps for operating the autoinjector medical device.

### DETAILED DESCRIPTION OF THE PREFERRED EMBODIMENTS

Certain terminology is used in the following description for convenience only and is not limiting. The words "front", "rear", "upper", and "lower" designate directions in the drawings to which reference is made. The words "inwardly" and "outwardly" refer to directions towards and away from parts referenced in the drawings. "Axially" refers to a direction along the axis of an axle, shaft, pin, or the like. A reference to a list of items that are cited as "at least one of a, b, or c" (where a, b, and c represent the items being listed) means any single one of the items a, b, or c, or combinations thereof are included. The terms "about" and "approximately" encompass +/- 10% of an indicated value unless otherwise noted. The term "generally" in connection with a radial direction encompasses +/- 25 degrees. The terminology includes the words specifically noted above, derivatives thereof, and words of similar import.

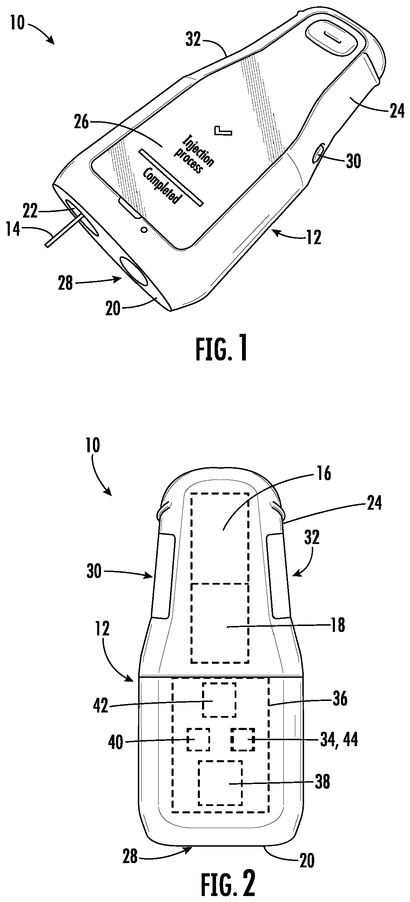

FIG. 1 is a perspective front-view of an autoinjector medical device 10 according to the present invention. FIG. 2 is a rear view of the autoinjector medical device 10, illustrating internal componentry of the autoinjector medical device 10. FIGS. 1-2 will be discussed together. In addition, hereinafter the autoinjector medical device 10 will be referred to as the "device 10", but it is to be understood that the autoinjector medical device 10 and the device 10 are referring to the same assembly.

The device 10 is configured to deliver a dose of a particular drug or medication into a user, thereby making it easier to self-administer needle-based drugs and medication. Further, the device 10 utilizes one-use, disposable cassettes and syringes (not shown) that are prefilled with the drug or medication to be delivered. After the drug or medication has been administered, the cassette and the syringe can be disposed of and a new prefilled cassette and syringe can be inserted into the device 10 for the next drug delivery.

As illustrated in FIGS. 1-2, the device 10 includes at least a cover 12, a needle 14, a power source 16, and an actuator 18. The cover 12 is configured to surround and protect the other components of the device 10 that are positioned within the cover 12 of the device 10. The power source 16 can be positioned within the cover 12 and the power source 16 can be utilized to provide electrical energy to the actuator 18, which is also positioned within the cover 12 of the device 10. In some examples, the power source 16 can be a battery. In other examples, the power source 16 can be an electrical cable and plug that is electrically coupled to a source of electrical energy that is outside and separate from the cover 12 and the overall device 10. In either example, the power source 16 is configured to provide electrical energy to the actuator 18 to cause administration of the drug upon the user pressing a button disposed on or adjacent the cover 12. In addition, the power source 16 is configured to provide electrical energy to the overall device 10 to power each of the electrical components of the device 10.

The actuator 18 can be coupled, directly or indirectly, to the needle 14, and the actuator 18 can cause the needle 14 to translate in and out of the cover 12 to administer the drug. As will be appreciated by a person having ordinary skill in the art, the needle 14 is configured to be inserted into a user's body for delivery of the drug into the user's body. As such, in operation, a user can press a button or switch (not shown) to activate the power source 16 which transfers electrical energy to the actuator 18. In turn, this causes the needle 14 to translate out from within the cover 12 such that the needle 14 enters the user's body to administer and dispense the drug or medication from the syringe (not shown) within the device 10 into the user's body.

The cover 12 can include an engaging surface 20 disposed at or on a bottom surface of the cover 12 of the device 10. In other words, the engaging surface 20 is the surface of the cover 12 and/or the device 10 that is directly pressed against a user's body during the injection of the drug or medication. The engaging surface 20 can include a needle outlet 22 disposed on or extending through the engaging surface 20 of the cover 12. In some examples, the needle outlet 22 can be an aperture that extends through the engaging surface 20 of the cover 12, allowing the needle 14 to translate in and out of the cover 12 through the needle outlet 22.

In addition, the cover 12 can include a handle 24 that is formed in the cover 12. In some examples, the handle 24 can be formed integral with the cover 12. The handle 24 provides a narrowed portion/width of the cover 12 in which a user can grasp and hold onto during use of the device 10. As illustrated, the handle 24 is disposed at an opposite end of the cover 12 and the engaging surface 20. Therefore, a user can grasp the handle 24 at one end of the cover 12 and the device 10, and the user can press the engaging surface 20 directly onto a skin surface of the user at the opposite end of the cover 12 and the device 10. As illustrated best in FIG. 1, the device 10 can also include a display 26 disposed on or coupled to the cover 12. In some examples, the display 26 can be a graphical display that is configured to provide a visual presentation of the data collected by the device 10, discussed further below.

In some examples, the device 10 can include a first sensor electrode 28, a second sensor electrode 30, and a third electrode 32. In other examples, the device 10 may only include the first sensor electrode 28 and the second sensor electrode 30. The first sensor electrode 28 can be disposed on or adjacent the engaging surface 20, and the first sensor electrode 28 can be disposed adjacent the needle outlet 22. The second sensor electrode 30 can be disposed on or extend through the cover 12, and the second sensor electrode 30 can be disposed on the handle 24 of the cover 12. The first sensor electrode 28 and the second sensor electrode 30 can each be electrode sensors that are configured to measure and record electrical signals produced by a heart and/or the body of a user. More specifically, the first sensor electrode 28 and the second sensor electrode 30 can each be electrode sensors that are configured to measure and record electrical signals produced by the heart and/or body of a user before, during, and after insertion of the needle 14 and dispensing of the drug or medication into the user.

As such, the first sensor electrode 28 and the second sensor electrode 30 can be utilized for real-time monitoring of physiological parameters at or adjacent the point of care (i.e., needle 14 insertion), providing the patient and/or physician health related data regarding the health of the patient. More specifically, the first sensor electrode 28 and the second sensor electrode 30 of the device 10 can be utilized for electrocardiogram (ECG) sensing of the user/patient, and the ECG measurement data and physiological parameters measured and computed by the device 10 can be shared to the patient's physician for remote patient monitoring, discussed further below.

In some examples, the first sensor electrode 28 and the second sensor electrode 30 can be configured to measure and record one or more of heart rate, heart rate variability, physiological stress, energy expenditure, atrial fibrillation, and arrhythmia of the user before, during, and after insertion of the needle 14 and dispensing of the drug/medication into the user. In other examples, the first sensor electrode 28 and the second sensor electrode 30 can be configured for bio-electrical impedance sensing of the user, and the bio-impedance measurement data and physiological parameters measured and computed by the device 10 can be shared to a physician for remote patient monitoring. More specifically, the first sensor electrode 28 and the second sensor electrode 30 can be configured to perform bio-impedance analysis (BIA), estimate body composition (e.g., body fat mass), and hydration level (e.g., total body water) of the user.

The data collected by the first sensor electrode 28 and the second sensor electrode 30 of the device 10 can then be presented on the display 26 of the device 10 for the user to view. Further, the data collected by the device 10 can also be transmitted by the device 10 to the patient's physician or a device that is separate and remote from the device 10. More specifically, the device 10 can include an input-output device 34 that is positioned within the cover 12 of the device 10 and electrically coupled to the power source 16 of the device 10. The input-output device 34 can be configured to transfer data collected by the device 10 to a device separate and remote from the device 10, such as a device remotely accessible by the patient's physician. In some examples, the input-output device 34 can transfer data collected by the device 10 through one or more of a wireless network protocol (Wi-Fi), a cellular signal, a Bluetooth standard, and a cloud-based data transfer service, among other options not specifically listed.

Further, in some examples, the device 10 can include stored data relating to the identity or biometric authentication of the user. The first sensor electrode 28 and the second sensor electrode 30 can be utilized to compare sensed parameters of the user (or the person holding the device 10) to the stored identity or biometric authentication data of the intended user to verify the identity of the user before injecting the drug or medication into the user. The device 10 including biometric authentication can increase the safety of the device 10 and prevent a drug or medication from being injected into anyone other than the intended user. For example, biometric authentication of the device 10 can prevent a child from accidently injecting themselves with a drug or medication that was not intended for their use.

As discussed, in some examples the device 10 can include only the first sensor electrode 28 and the second sensor electrode 30. In other examples, the device 10 can include the first sensor electrode 28, the second sensor electrode 30, and the third electrode 32. As illustrated in FIGS. 1-2, in some examples the third electrode 32 can be disposed on or extend through the cover 12, and the third electrode 32 can be disposed on the handle 24 of the cover 12. In such an example, the third electrode 32 can be positioned on an opposite side of the handle 24 as the second sensor electrode 30, such that when a user grasps the handle 24 their hand will contact both the second sensor electrode 30 and the third electrode 32. In other examples, the third electrode 32 can be disposed on or adjacent the engaging surface 20, and the third electrode 32 can be disposed adjacent the needle outlet 22 and/or the first sensor electrode 28. In such an example, when the user presses the engaging surface 20 against a skin surface of their body, both the first sensor electrode 28 and the third electrode 32 will be directly in contact with a skin surface of their body.

In either example, the third electrode 32 can be a right-leg drive electrode that is configured to implement common-mode powerline interference suppression. In other words, the third electrode 32 can be utilized to determine and then cancel out natural frequencies/signals that are produced by the human body, which can cause interference and incorrect measurements and/or data during collection of the ECG measurements. As such, the third electrode 32 is not required but is advantageous and desirable because it results in more accurate measurements of the physiological parameters of the user's heart and/or body by the device 10 during collection of the ECG measurements. More specifically, the first sensor electrode 28, the second sensor electrode 30, and the third electrode 32 can gather information and data related to the user (e.g., ECG measurements, natural frequencies/signals, etc.), and then the data can be processed by algorithms to produce accurate results relating to the many different properties/characteristics of the user's heart and overall health. In some examples, the algorithms can be stored within the device 10 for processing by the device 10. In other examples, the algorithms can be stored on a device that is separate from the device 10, such that the data is processed after being transferred from the device 10 to a separate and remote device/controller.

It is to be understood that in an embodiment in which the device 10 is utilized for bio-impedance measurements, the third electrode 32 may not be included in the device 10 and/or the third electrode 32 may not be activated or utilized during the bio-impedance measurements. The aforementioned is due to the fact that the right-leg drive electrode common-mode powerline interference suppression (facilitated by the third electrode 32) is not utilized during bio-impedance measurements, as will be appreciated by those skilled in the art.

As illustrated in FIG. 2, the device 10 can further include a circuit board 36 including an analog front-end stage 38, an analog to digital converter 40, a microprocessor 42, an alarm 44, and the input-output device 34. Each of the aforementioned components can be electrically and/or communicatively coupled to the circuit board 36 for transferring electrical power and data, and the circuit board 36 can be electrically coupled to the power source 16 for receiving electrical energy. The analog front-end stage 38 can be an electrical component coupled to the circuit board 36 within the device 10. The analog front-end stage 38 can be configured to precondition the electrical signals measured and recorded by the first sensor electrode 28 and the second sensor electrode 30 before processing and outputting results regarding the electrical signals. In addition, the analog front-end stage 38 can perform analog filtering of the signals gathered by the sensors 28, 30 to remove the common-mode powerline interference, which could be capacitively coupled by patient's body. The analog front-end stage 38 also presents the sensors 28, 30 with very-high input impedance to avoid ECG signal attenuation by design.

In some examples, the analog to digital converter 40 can also be an electrical component coupled to the circuit board 36 within the device 10. In other examples, the analog to digital converter 40 can be embedded directly into the microprocessor 42. In either example, the analog to digital converter 40 can be configured to sample the measured ECG and other electrical signals from the sensors 28, 30, and convert the signals into digital data so that the data can be processed by the microprocessor 42. The microprocessor 42 can be an electrical component coupled to the circuit board 36 within the device 10. The microprocessor 42 can be configured to implement additional filtering in the digital domain for higher signalto-noise ratio, and configured to perform ECG digital signal processing to analyze the characteristics of the ECG waveform and compute the corresponding physiological parameters. In addition, the microprocessor 42 can also be utilized to determine when there is sufficient contact between the electrodes (sensor electrodes 28, 30, and third electrode 32) and the user's skin surface. This lead on/off information can be used by the microprocessor 42 to determine if input data samples received by the device 10 are valid before executing further processing.

The alarm 44 can be an electrical component coupled to the circuit board 36 within the device 10. The alarm 44 can be configured to alert the user when the measured and recorded electrical signals exceed a pre-defined threshold limit. More specifically, the device 10 can include stored pre-defined limits for specific physiological parameters of the heart of the user. The device 10 can compare the measured and recorded electrical signals of the heart to the pre-defined threshold limits, and if the measured and recorded electrical signals of the heart exceed the pre-defined threshold limits, the device 10 can prevent the insertion of the needle 14 and the dispensing of the drug into the user. In one example, a pre-defined threshold (maximum) heartrate can be stored within the device 10, and if the measured/recorded heartrate of the user exceeds the pre-defined threshold limit, the device 10 can prevent the insertion of the needle 14 and the dispensing of the drug into the user. In addition, the alarm 44 could be a visual notification that is displayed on the display 26, a light that turns on to indicate an issue, or an audio notification, among other options not specifically listed. The alarm 44 is a safety feature of the device 10 that is configured to prevent harm to the user of the device 10.

As discussed, the device 10 can include a display 26 and the display 26 can be utilized to visually present the user with real-time ECG waveform information and other bio-electrical information. In addition, the display 26 can visually present data related to other physiological parameters of the user's heart and/or body that were sensed by the sensor electrodes 28, 30 and computed by the microprocessor 42 and other components of the circuit board 36, such as bio-impedance analysis (BIA) data, estimated body composition (e.g., body fat content), hydration level, etc. The device 10 can be enabled to perform bio-electrical (ECG, BIA, etc.) measurements before, during, and after the occurrence of an injection of a drug or medication into the user. In addition, the bio-electrical measurements obtained by the device 10 can be utilized as a feature itself of the device 10, without necessarily having to be associated with the execution of an injection of a drug or medication into the user. As such, in some examples, the device 10 can be utilized to gather and obtain bio-electrical measurements of the user for monitoring the user's health without injecting a drug or medication into the user.

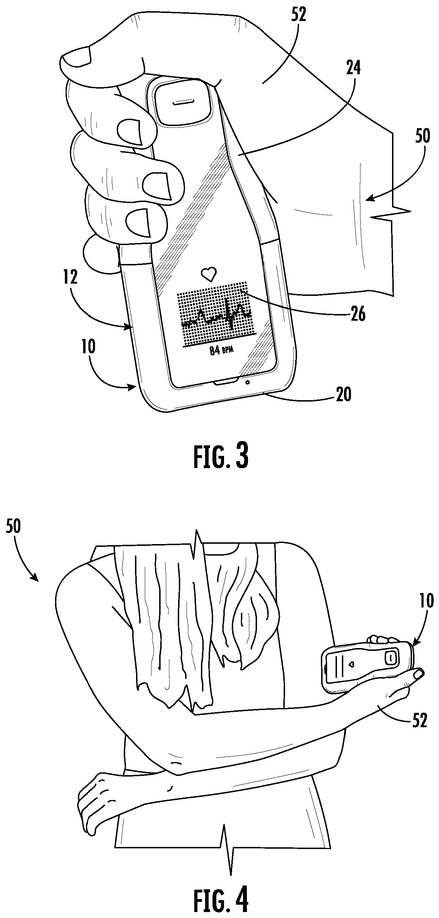

FIG. 3 is a perspective view of a user 50 holding and operating the device 10. FIG. 4 is another view of the user 50 holding and operating the device 10. FIGS. 3-4 will be discussed together. To collect electrical signals of the heart and/or body of the user 50 with the device 10, the user 50 grasps the handle 24 of the device 10 with a hand 52 on a first side of the user's body, such that during the grasping the second sensor electrode 30 of the device 10 directly contacts a skin surface of the hand 52 of the user 50. Then the user 50 presses the engaging surface 20 of the device 10 directly against a skin surface of a portion (e.g., a leg, an arm, etc.) of the user's body on a second side of the user's body, such that during the pressing the first sensor electrode 28 of the device 10 directly contacts a skin surface of the portion (e.g., a leg, an arm, etc.) of the second side of the user's body.

It is to be understood that the first side of the user's body and the second side of the user's body are positioned on opposite sides of a sagittal plane (i.e., a plane that divides the body into right and left sides) of the user's body. Further, it is to be understood that with the device 10 held in a hand on one side of the body, the engaging surface 20 must contact the other or opposite side of the body, such that the signal produced by the device 10 passes through the heart of the user 50 to gather information relating to the heart (ECG measurements). Therefore, if the device 10 is held within the right hand of the user 50, then the engaging surface 20 must directly contact a skin surface on the left side of the body (i.e., left side of the heart). If the device 10 is held within the left hand of the user 50, then the engaging surface 20 must directly contact a skin surface on the right side of the body (i.e. right side of the heart). The previously described use/orientation is necessary to gather the desired information about the heart of the user 50.

Next, the device 10 can utilize the first sensor electrode 28 and the second sensor electrode 30 to measure and collect electrical signals produced by the heart and/or body of the user 50. The analog front-end stage 38 can process the measured and collected electrical signals, with the processing including filtering the electrical signals to remove common-mode powerline interferences by leveraging the use of the third electrode 32 (for ECG measurements, but not for BIA measurements). The analog to digital converter 40 can then convert the processed electrical signals and output digital data that can be processed by the microprocessor 42. The microprocessor 42 can then output physiological parameters or ECG and BIA measurements relating to the heart and/or body of the user 50. The physiological parameters or ECG measurements can be one or more of heart rate, heart rate variability, physiological stress, energy expenditure, atrial fibrillation, and arrhythmia of the user, among other properties/characteristics not specifically disclosed. In addition, the physiological parameters can include sensed bio-impedance analysis (BIA) data, estimated body composition (e.g., body fat content), and hydration level of the user (e.g., total body water).

Then the device 10 can display the physiological parameters on the display 26 of the device 10. In addition, the device 10 can also communicate the physiological parameters of the user's heart and/or body through the input-output device 34 of the device 10 to a device/system that is separate and remote from the device 10, in which a physician can remotely access data to view and analyze the data collected by the device 10. As such, the device 10 can remotely gather data relating to a user's heart and/or body, and send the data to a physician for analysis, which can be utilized to identify potentially serious illness, check vital conditions, and/or the heart condition of the user or patient.

The device 10 of the present application provides many advantages that will be appreciated by a person having ordinary skill in the art. For example, home-therapies that require self-administration of drugs which may produce effects on the cardiac cycle could demand frequent monitoring of the patient's ECG signal. The integration of an ECG subsystem (first sensor electrode 28, second sensor electrode 30, circuit board 36, etc.) in the device 10 enables the patient to execute ECG measurements from home using the same device 10 that is used for injection, without necessarily having to seek assistance from professional personnel. The patient's physician could prescribe the patient to execute contextual ECG measurements during the therapy to monitor the physiological parameters of the patient in response to the injection-based treatment. In addition, the sensed bio-impedance analysis (BIA) data can be evaluated by a physician to aid in patient weight reduction and control due to the device 10 being capable of measuring bio-parameters such as body mass index (BMI), body fat content, hydration (water) level, etc. of the user. Convenient sensor electrodes (i.e., the first sensor electrode 28 and the second sensor electrode 30) placement along the device 10 eases and simplifies the measurement process for the patient.

In addition, the remotely connected device 10 enables remote patient monitoring to present the physician with additional datasets to obtain a clearer picture on the patient's general health. This allows the physician to deliver better quality of care for the patient. Although the device 10 of the present application does not produce the same amount of data as a twelve-lead ECG measurement device that is exclusive to a clinical setting, the device 10 provides many medical insights that can be extracted by dedicated data processing algorithms (within the device 10 or separate from the device 10). For example, the device 10 can measure and record heart rate, heart rate variability, physiological stress, energy expenditure, atrial fibrillation, arrhythmia of the patient, among other physiological parameters not specifically listed. In addition, the device 10 can be configured to perform bio-impedance analysis (BIA), estimate body composition (e.g., body fat content), and hydration level of the user. The patient's physician can use the real-time data to discover patterns and potentially identify early on possible chronic health conditions. As such, the device 10 of the present application provides many advantages that are not currently provided by traditional or standard autoinjector medical devices.

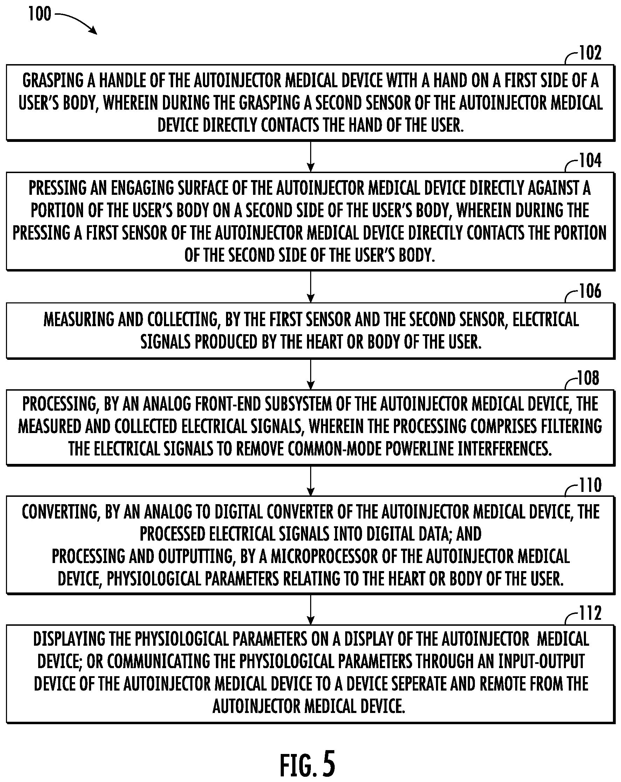

FIG. 5 is a schematic block diagram illustrating a method 100 for operating the device 10. As illustrated, in some examples the method 100 can include method steps 102 - 112. In other examples, the method 100 can include more or less steps than illustrated in FIG. 5 and discussed below. The method 100 can be for collecting electrical signals of a heart and/or body of a user 50 with an autoinjector medical device 10.

Step 102 can include grasping the handle 24 of the device 10 with a hand 52 on a first side of a user's body, wherein during the grasping the second sensor electrode 30 of the device 10 directly contacts the hand 52 of the user 50. Step 104 can include pressing the engaging surface 20 of the device 10 directly against a portion of the user's body on a second side of the user's body, wherein during the pressing the first sensor electrode 28 of the device 10 directly contacts the portion of the second side of the user's body. Step 106 can include measuring and collecting, by the first sensor electrode 28 and the second sensor electrode 30, electrical signals produced by the heart and/or body of the user 50. Step 108 can include processing, by the analog front-end stage 38 of the device 10, the measured and collected electrical signals, wherein the processing comprises filtering the electrical signals to remove common-mode powerline interferences. Step 110 can include converting, by the analog to digital converter 40 of the device 10, the processed electrical signals into digital data, and further processing and outputting, by the microprocessor 42 of the device 10, physiological parameters relating to the heart and/or body of the user 50. Step 112 can include displaying the physiological parameters on the display 26 of the device 10, and/or communicating the physiological parameters through the input-output device 34 of the device 10 to a device separate and remote from the autoinjector medical device 10. As discussed, in some examples the method 100 can include more or less than steps 102 - 112, previously discussed.

Having thus described the present embodiments in detail, it is to be appreciated and will be apparent to those skilled in the art that many physical changes, only a few of which are exemplified in the detailed description of the disclosure, could be made without altering the inventive concepts and principles embodied therein.

It is also to be appreciated that numerous embodiments incorporating only part of the preferred embodiment are possible which do not alter, with respect to those parts, the inventive concepts and principles embodied therein. The present embodiment and optional configurations are therefore to be considered in all respects as exemplary and/or illustrative and not restrictive, the scope of the disclosure being indicated by the appended claims rather than by the foregoing description, and all alternate embodiments and changes to this embodiment which come within the meaning and range of equivalency of said claims are therefore to be embraced therein.

## Claims

1. An autoinjector medical device (10) comprising:
a cover (12) including:
an engaging surface (20) including a needle outlet (22); and
a handle (24) formed in the cover (12) at an opposite end of the cover (12) as the engaging surface (20);
a needle (14) configured to translate through the needle outlet (22);
a first sensor electrode (28) disposed on the engaging surface (20) and adjacent the needle outlet (22); and
a second sensor electrode (30) disposed on the handle (24),
wherein the first sensor electrode (28) and the second sensor electrode (30) are each electrode sensors configured to measure and record electrical signals produced by a heart or body of a user (50); and
wherein the first sensor electrode (28) and the second sensor electrode (30) are further configured for biometric authentication of the user (50), the device (10) further comprising stored identity data and being configured to compare sensed parameters of the user (50) to the stored identity data to verify the identity of the user (50) before allowing an injection.

2. The autoinjector medical device (10) of claim 1, further comprising a third electrode (32) disposed on the handle.

3. The autoinjector medical device of claim 2, wherein the third electrode (32) is positioned on an opposite side of the handle (24) as the second sensor electrode (30); or
wherein the third electrode (32) is a right-leg drive electrode configured to implement common-mode powerline interference suppression.

4. The autoinjector medical device of claim 1, wherein the first sensor electrode (28) and the second sensor electrode (30) are configured to measure and record electrical signals produced by the heart of the user (50) before, during, or after insertion of the needle (14) into the user (50); or
wherein the first sensor electrode (28) and the second sensor electrode (30) are configured to measure and record one or more of heart rate, heart rate variability, physiological stress, energy expenditure, atrial fibrillation, arrhythmia, body fat content, and hydration level in the user (50).

5. The autoinjector medical device of claim 1, further comprising an alarm (44) configured to alert the user (50) when the measured and recorded electrical signals exceed a pre-defined threshold limit; or
further comprising an analog front-end stage (38) configured to precondition the electrical signals measured and recorded by the first sensor electrode (28) and the second sensor electrode (30) before processing and outputting results regarding the electrical signals.

6. The autoinjector medical device of claim 1, wherein the autoinjector medical device (10) is an electro-mechanical autoinjector medical device including a power source (16) configured to supply electrical energy to an actuator (18), the actuator being coupled to the needle (14) and the actuator (18) being configured to cause the needle (14)to translate through the needle outlet (22).

7. The autoinjector medical device of claim 1, further comprising a display (26) disposed on or coupled to the cover (12), the display (26) being configured to provide a visual presentation of data collected by the autoinjector medical device (10).

8. The autoinjector medical device of claim 1, further comprising an input-output device (34) configured to transfer data collected by the autoinjector medical device (10) to a device separate and remote from the autoinjector medical device (10).

9. The autoinjector medical device of claim 8, wherein the input-output device (34) transfers collected data through one or more of a wireless network protocol (Wi-Fi), a cellular signal, a Bluetooth standard, and a cloud-based data transfer service.

10. A method of collecting electrical signals of a heart or body of a user (50) with an autoinjector medical device (10), the method comprising:
grasping a handle (24) of the autoinjector medical device (10) with a hand (52) on a first side of a user's body, wherein during the grasping a second sensor electrode (30) of the autoinjector medical device (10) directly contacts the hand (52) of the user (50);
pressing an engaging surface (20) of the autoinjector medical device (10) directly against a portion of the user's body on a second side of the user's body, wherein during the pressing a first sensor electrode (28) of the autoinjector medical device (10) directly contacts the portion of the second side of the user's body;
measuring and collecting, by the first sensor electrode and the second sensor electrode (28), electrical signals produced by the heart or body of the user (50); and
comparing the collected electrical signals to stored identity data to verify the identity of the user (50); and allowing an injection of a drug from the autoinjector medical device (10) only upon verification of the identity of the user (50).

11. The method of claim 10, further comprising directly contacting the first sensor electrode (28) and the second sensor electrode (30) to skin surfaces of the user's body.

12. The method of claim 11, wherein the first side of the user's body and the second side of the user's body are positioned on opposite sides of a sagittal plane of the user's body.

13. The method of claim 11, further comprising processing, by an analog front-end stage (38) of the autoinjector medical device (10), the measured and collected electrical signals, wherein the processing comprises filtering the electrical signals to remove common-mode powerline interferences.

14. The method of claim 13, further comprising:
converting, by an analog to digital converter (40) of the autoinjector medical device (10), the processed electrical signals into digital data; and
processing and outputting, by a microprocessor (42) of the autoinjector medical device (10), physiological parameters relating to the heart or body of the user (50).

15. The method of claim 14, further comprising:
displaying the physiological parameters on a display (26) of the autoinjector medical device (10); or
communicating the physiological parameters through an input-output device (34) of the autoinjector medical device (10) to a device separate and remote from the autoinjector medical device (10).

## Patentansprüche

1. Medizinische Autoinjektor-Vorrichtung (10), umfassend:
eine Abdeckung (12), beinhaltend:
eine Kontaktfläche (20), die einen Nadelaustritt (22) beinhaltet; und
einen Griff (24), der in der Abdeckung (12) an einem der Kontaktfläche (20) entgegengesetzten Ende der Abdeckung (12) ausgebildet ist;
eine Nadel (14), die konfiguriert ist, um sich durch den Nadelaustritt (22) zu verschieben;
eine erste Sensorelektrode (28), die auf der Kontaktfläche (20) und benachbart zum Nadelaustritt (22) angeordnet ist; und
eine zweite Sensorelektrode (30), die auf dem Griff (24) angeordnet ist,
wobei die erste Sensorelektrode (28) und die zweite Sensorelektrode (30) jeweils Elektrodensensoren sind, die konfiguriert sind, um elektrische Signale zu messen und aufzuzeichnen, die von einem Herz oder Körper eines Benutzers (50) erzeugt werden; und
wobei die erste Sensorelektrode (28) und die zweite Sensorelektrode (30) ferner zur biometrischen Authentifizierung des Benutzers (50) konfiguriert sind, wobei die Vorrichtung (10) ferner gespeicherte Identitätsdaten umfasst und konfiguriert ist, um erfasste Parameter des Benutzers (50) mit den gespeicherten Identitätsdaten zu vergleichen, um die Identität des Benutzers (50) zu verifizieren, bevor eine Injektion zugelassen wird.

2. Medizinische Autoinjektor-Vorrichtung (10) nach Anspruch 1, ferner umfassend eine dritte Elektrode (32), die auf dem Griff angeordnet ist.

3. Medizinische Autoinjektor-Vorrichtung nach Anspruch 2, wobei die dritte Elektrode (32) auf einer der zweiten Sensorelektrode (30) gegenüberliegenden Seite des Griffs (24) positioniert ist; oder
wobei die dritte Elektrode (32) eine Right-Leg-Drive-Elektrode ist, die konfiguriert ist, um eine Gleichtakt-Netzstörungsunterdrückung zu implementieren.

4. Medizinische Autoinjektor-Vorrichtung nach Anspruch 1, wobei die erste Sensorelektrode (28) und die zweite Sensorelektrode (30) konfiguriert sind, um elektrische Signale zu messen und aufzuzeichnen, die vom Herz des Benutzers (50) vor, während oder nach dem Einführen der Nadel (14) in den Benutzer (50) erzeugt werden; oder
wobei die erste Sensorelektrode (28) und die zweite Sensorelektrode (30) konfiguriert sind, um eines oder mehrere von Herzfrequenz, Herzfrequenzvariabilität, physiologischem Stress, Energieverbrauch, Vorhofflimmern, Arrhythmie, Körperfettanteil und Hydratationszustand des Benutzers (50) zu messen und aufzuzeichnen.

5. Medizinische Autoinjektor-Vorrichtung nach Anspruch 1, ferner umfassend einen Alarm (44), der konfiguriert ist, um den Benutzer (50) zu warnen, wenn die gemessenen und aufgezeichneten elektrischen Signale einen vordefinierten Schwellenwert überschreiten; oder
ferner umfassend eine analoge Eingangsstufe (38), die konfiguriert ist, um die von der ersten Sensorelektrode (28) und der zweiten Sensorelektrode (30) gemessenen und aufgezeichneten elektrischen Signale vorzubereiten, bevor Ergebnisse bezüglich der elektrischen Signale verarbeitet und ausgegeben werden.

6. Medizinische Autoinjektor-Vorrichtung nach Anspruch 1, wobei die medizinische Autoinjektor-Vorrichtung (10) eine elektromechanische medizinische Autoinjektor-Vorrichtung ist, die eine Energiequelle (16) beinhaltet, welche konfiguriert ist, um einen Aktuator (18) mit elektrischer Energie zu versorgen, wobei der Aktuator mit der Nadel (14) gekoppelt ist und der Aktuator (18) konfiguriert ist, um zu bewirken, dass sich die Nadel (14) durch den Nadelaustritt (22) verschiebt.

7. Medizinische Autoinjektor-Vorrichtung nach Anspruch 1, ferner umfassend eine Anzeige (26), die auf der Abdeckung (12) angeordnet oder mit dieser gekoppelt ist, wobei die Anzeige (26) konfiguriert ist, um eine visuelle Darstellung von Daten bereitzustellen, die von der medizinischen Autoinjektor-Vorrichtung (10) gesammelt wurden.

8. Medizinische Autoinjektor-Vorrichtung nach Anspruch 1, ferner umfassend eine Eingabe-Ausgabe-Vorrichtung (34), die konfiguriert ist, um von der medizinischen Autoinjektor-Vorrichtung (10) gesammelte Daten an eine von der medizinischen Autoinjektor-Vorrichtung (10) separate und entfernte Vorrichtung zu übertragen.

9. Medizinische Autoinjektor-Vorrichtung nach Anspruch 8, wobei die Eingabe-Ausgabe-Vorrichtung (34) gesammelte Daten durch eines oder mehrere von einem drahtlosen Netzwerkprotokoll (Wi-Fi), einem Mobilfunksignal, einem Bluetooth-Standard und einem cloudbasierten Datenübertragungsdienst überträgt.

10. Verfahren zum Sammeln elektrischer Signale eines Herzes oder Körpers eines Benutzers (50) mit einer medizinischen Autoinjektor-Vorrichtung (10), wobei das Verfahren umfasst:
Ergreifen eines Griffs (24) der medizinischen Autoinjektor-Vorrichtung (10) mit einer Hand (52) auf einer ersten Seite eines Körpers eines Benutzers, wobei während des Ergreifens eine zweite Sensorelektrode (30) der medizinischen Autoinjektor-Vorrichtung (10) die Hand (52) des Benutzers (50) direkt kontaktiert;
Drücken einer Kontaktfläche (20) der medizinischen Autoinjektor-Vorrichtung (10) direkt gegen einen Abschnitt des Körpers des Benutzers auf einer zweiten Seite des Körpers des Benutzers, wobei während des Drückens eine erste Sensorelektrode (28) der medizinischen Autoinjektor-Vorrichtung (10) den Abschnitt der zweiten Seite des Körpers des Benutzers direkt kontaktiert;
Messen und Sammeln elektrischer Signale, die vom Herz oder Körper des Benutzers (50) erzeugt werden, durch die erste Sensorelektrode und die zweite Sensorelektrode (28); und
Vergleichen der gesammelten elektrischen Signale mit gespeicherten Identitätsdaten, um die Identität des Benutzers (50) zu verifizieren; und Zulassen einer Injektion eines Medikaments aus der medizinischen Autoinjektor-Vorrichtung (10) erst nach Verifizierung der Identität des Benutzers (50).

11. Verfahren nach Anspruch 10, ferner umfassend das direkte Kontaktieren der ersten Sensorelektrode (28) und der zweiten Sensorelektrode (30) mit Hautoberflächen des Körpers des Benutzers.

12. Verfahren nach Anspruch 11, wobei die erste Seite des Körpers des Benutzers und die zweite Seite des Körpers des Benutzers auf gegenüberliegenden Seiten einer Sagittalebene des Körpers des Benutzers positioniert sind.

13. Verfahren nach Anspruch 11, ferner umfassend das Verarbeiten der gemessenen und gesammelten elektrischen Signale durch eine analoge Eingangsstufe (38) der medizinischen Autoinjektor-Vorrichtung (10), wobei das Verarbeiten das Filtern der elektrischen Signale umfasst, um Gleichtakt-Netzstörungen zu entfernen.

14. Verfahren nach Anspruch 13, ferner umfassend:
Umwandeln der verarbeiteten elektrischen Signale in digitale Daten durch einen Analog-Digital-Wandler (40) der medizinischen Autoinjektor-Vorrichtung (10); und
Verarbeiten und Ausgeben physiologischer Parameter bezüglich des Herzes oder Körpers des Benutzers (50) durch einen Mikroprozessor (42) der medizinischen Autoinjektor-Vorrichtung (10) .

15. Verfahren nach Anspruch 14, ferner umfassend:
Anzeigen der physiologischen Parameter auf einer Anzeige (26) der medizinischen Autoinjektor-Vorrichtung (10); oder
Kommunizieren der physiologischen Parameter durch eine Eingabe-Ausgabe-Vorrichtung (34) der medizinischen Autoinjektor-Vorrichtung (10) an eine von der medizinischen Autoinjektor-Vorrichtung (10) separate und entfernte Vorrichtung.

## Revendications

1. Dispositif médical auto-injecteur (10) comprenant :
un couvercle (12) comportant :
une surface de contact (20) comportant une sortie d'aiguille (22) ; et
une poignée (24) formée dans le couvercle (12) à une extrémité opposée du couvercle (12) par rapport à la surface de contact (20) ;
une aiguille (14) configurée pour se déplacer en translation à travers la sortie de l'aiguille (22) ;
une première électrode de capteur (28) disposée sur la surface de contact (20) et adjacente à la sortie de l'aiguille (22) ; et
une seconde électrode de capteur (30) disposée sur la poignée (24),
dans lequel la première électrode de capteur (28) et la seconde électrode de capteur (30) sont chacune des électrodes de capteur configurées pour mesurer et enregistrer les signaux électriques produits par le cœur ou le corps d'un utilisateur (50) ; et
dans lequel la première électrode de capteur (28) et la seconde électrode de capteur (30) sont en outre configurées pour l'authentification biométrique de l'utilisateur (50), le dispositif (10) comprenant en outre des données d'identité stockées et étant configuré pour comparer les paramètres détectés de l'utilisateur (50) aux données d'identité stockées afin de vérifier l'identité de l'utilisateur (50) avant d'autoriser une injection.

2. Dispositif médical auto-injecteur (10) de la revendication 1, comprenant en outre une troisième électrode (32) disposée sur la poignée.

3. Dispositif médical auto-injecteur de la revendication 2, dans lequel la troisième électrode (32) est positionnée sur un côté opposé de la poignée (24) par rapport à la seconde électrode de capteur (30) ; ou
dans lequel la troisième électrode (32) est une électrode de commande de jambe droite configurée pour mettre en œuvre la suppression des interférences de mode commun du réseau électrique.

4. Dispositif médical auto-injecteur de la revendication 1, dans lequel la première électrode de capteur (28) et la seconde électrode de capteur (30) sont configurées pour mesurer et enregistrer les signaux électriques produits par le cœur de l'utilisateur (50) avant, pendant ou après l'insertion de l'aiguille (14) dans l'utilisateur (50) ; ou
dans lequel la première électrode de capteur (28) et la seconde électrode de capteur (30) sont configurées pour mesurer et enregistrer l'un ou plusieurs des paramètres suivants chez l'utilisateur (50) : fréquence cardiaque, variabilité de la fréquence cardiaque, stress physiologique, dépense énergétique, fibrillation auriculaire, arythmie, teneur en graisse corporelle et niveau d'hydratation.

5. Dispositif médical auto-injecteur de la revendication 1, comprenant en outre une alarme (44) configurée pour alerter l'utilisateur (50) lorsque les signaux électriques mesurés et enregistrés dépassent un seuil prédéfini ; ou
comprenant en outre un étage d'entrée analogique (38) configuré pour préconditionner les signaux électriques mesurés et enregistrés par la première électrode de capteur (28) et la seconde électrode de capteur (30) avant de traiter et de produire les résultats concernant les signaux électriques.

6. Dispositif médical auto-injecteur de la revendication 1, dans lequel le dispositif médical auto-injecteur (10) est un dispositif médical auto-injecteur électromécanique comportant une source d'alimentation (16) configurée pour fournir de l'énergie électrique à un actionneur (18), l'actionneur étant couplé à l'aiguille (14) et l'actionneur (18) étant configuré pour amener l'aiguille (14) à se déplacer en translation à travers la sortie de l'aiguille (22).

7. Dispositif médical auto-injecteur de la revendication 1, comprenant en outre un écran (26) disposé sur ou couplé au couvercle (12), l'écran (26) étant configuré pour fournir une présentation visuelle des données collectées par le dispositif médical auto-injecteur (10).

8. Dispositif médical auto-injecteur de la revendication 1, comprenant en outre un dispositif d'entrée-sortie (34) configuré pour transférer les données collectées par le dispositif médical auto-injecteur (10) vers un dispositif distinct et distant du dispositif médical auto-injecteur (10).

9. Dispositif médical auto-injecteur de la revendication 8, dans lequel le dispositif d'entrée-sortie (34) transfère les données collectées par l'intermédiaire de l'un ou plusieurs des éléments suivants : un protocole de réseau sans fil (Wi-Fi), un signal cellulaire, une norme Bluetooth et un service de transfert de données basé sur le cloud.

10. Procédé de collecte de signaux électriques du cœur ou du corps d'un utilisateur (50) à l'aide d'un dispositif médical auto-injecteur (10), le procédé comprenant :
la saisie d'une poignée (24) du dispositif médical auto-injecteur (10) avec une main (52) d'un premier côté du corps d'un utilisateur, dans lequel, pendant la saisie, une seconde électrode de capteur (30) du dispositif médical auto-injecteur (10) entre directement en contact avec la main (52) de l'utilisateur (50) ;
le fait de presser une surface de contact (20) du dispositif médical auto-injecteur (10) directement contre une partie du corps de l'utilisateur sur un second côté du corps de l'utilisateur, dans lequel, pendant la pression, une première électrode de capteur (28) du dispositif médical auto-injecteur (10) entre directement en contact avec la partie du second côté du corps de l'utilisateur ;
la mesure et la collecte, par la première électrode de capteur et la seconde électrode de capteur (28), des signaux électriques produits par le cœur ou le corps de l'utilisateur (50) ; et
la comparaison des signaux électriques collectés aux données d'identité stockées pour vérifier l'identité de l'utilisateur (50) ; et l'autorisation de l'injection d'un médicament à partir du dispositif médical auto-injecteur (10) uniquement après vérification de l'identité de l'utilisateur (50).

11. Procédé de la revendication 10, comprenant en outre le contact direct de la première électrode de capteur (28) et de la seconde électrode de capteur (30) avec les surfaces cutanées du corps de l'utilisateur.

12. Procédé de la revendication 11, dans lequel le premier côté du corps de l'utilisateur et le second côté du corps de l'utilisateur sont positionnés de part et d'autre d'un plan sagittal du corps de l'utilisateur.

13. Procédé de la revendication 11, comprenant en outre le traitement, par un étage d'entrée analogique (38) du dispositif médical auto-injecteur (10), des signaux électriques mesurés et collectés, dans lequel le traitement comprend le filtrage des signaux électriques pour supprimer les interférences de mode commun du réseau électrique.

14. Procédé de la revendication 13, comprenant en outre :
la conversion, par un convertisseur analogique-numérique (40) du dispositif médical auto-injecteur (10), des signaux électriques traités en données numériques ; et
le traitement et la production, par un microprocesseur (42) du dispositif médical auto-injecteur (10), des paramètres physiologiques relatifs au cœur ou au corps de l'utilisateur (50).

15. Procédé de la revendication 14, comprenant en outre :
l'affichage des paramètres physiologiques sur un écran (26) du dispositif médical auto-injecteur (10) ; ou
la communication des paramètres physiologiques par l'intermédiaire d'un dispositif d'entrée-sortie (34) du dispositif médical auto-injecteur (10) à un dispositif distinct et distant du dispositif médical auto-injecteur (10).
